# EUROPÄISCHE PATENTSCHRIFT

(11) **EP 1 921 993 B1**
(45) Veröffentlichungstag und Bekanntmachung des Hinweises auf die Patenterteilung: **28.09.2016**
(21) Anmeldenummer: 06777103.0
(22) Anmeldetag: 29.08.2006
(51) Int. Cl.: A61B 5/151

(54) **ANORDNUNG ZUM AUFNEHMEN VON KÖRPERFLÜSSIGKEITEN**
ASSEMBLY FOR RECEIVING BODY FLUIDS
DISPOSITIF POUR ABSORBER DES LIQUIDES CORPORELS

(30) Priorität: 01.09.2005 EP 05019055
(43) Veröffentlichungstag der Anmeldung: 21.05.2008
(73) Patentinhaber: F.Hoffmann-La Roche AG, 4070 Basel (CH); Roche Diabetes Care GmbH, 68305 Mannheim (DE)
(72) Erfinder: HAAR, Hans-Peter, 69168 Wiesloch (DE); CALASSO, Irio, Giuseppe, 6415 Arth (CH); FUERST, Otto, 68519 Viernheim (DE)
(74) Vertreter: Pfiz, Thomas
(86) Internationale Anmeldenummer: PCT/EP2006/008436
(87) Internationale Veröffentlichungsnummer: WO 2007/025713

(56) Entgegenhaltungen:
- EP-A- 1 284 121
- WO-A2-2005/084530

## Beschreibung

Die Erfindung betrifft eine Anordnung bzw. Einrichtung zum Aufnehmen von Körperflüssigkeiten wie Blut, mit einem vorzugsweise mit einem Stechorgan zum Einstechen in ein Körperteil versehenen Probenentnahmeelement, das einen Sammelbereich zum Sammeln von durch einen Einstich erhaltener Körperflüssigkeit aufweist, und mit einem über den Sammelbereich mit Körperflüssigkeit beaufschlagbaren, vorzugsweise mit einem Testfeld für einen Analyten in der Körperflüssigkeit versehenen Probenempfangselement. Die Erfindung betrifft weiter ein Verfahren zur Herstellung eines solchen Probenentnahmeelements.

In einer früheren Anmeldung WO-A1-2005/084545 der Anmelder ist eine Anordnung zum Sammeln von Körperflüssigkeit für Analysezwecke, speziell zur Blutglucose-Konzentrationsbestimmung beschrieben. Daraus geht ein Stechelement mit einer Sammelzone zum Aufnehmen von Körperflüssigkeit hervor, wobei die Sammelzone durch Lateralöffnungen gebildet sein kann. Der Flüssigkeitstransfer kann damit senkrecht zur Stechrichtung auf ein in fluidischen Kontakt gebrachtes Probenaufnahmeelement erfolgen, so dass ein kapillarer Transport über makroskopische Strecken mit hohem Totvolumen vermieden werden kann.

Ausgehend hiervon liegt der Erfindung die Aufgabe zugrunde, die im Stand der Technik bekannten Systeme weiter zu entwickeln und eine Anordnung der eingangs angegebenen Art im Sinne einer verbesserten Probenaufnahme zu optimieren, wobei ein Erfindungsziel auch in einer vereinfachten Herstellung besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine möglichst zuverlässige kapillare Probenaufnahme durch einen beidseitig offenen Kapillarschlitz zu erreichen. Dementsprechend wird erfindungsgemäß vorgeschlagen, dass der Sammelbereich durch einen als Kapillare langgestreckten, über Seitenöffnungen an dem Probenentnahmeelement beidseitig offenen Längsschlitz gebildet ist, wobei der Längsschlitz in einer Sammelstellung des Probenentnahmeelements von dem Probenempfangselement abgetrennt ist, so dass keine Körperflüssigkeit übertragen wird, und in einer Transferstellung des Probenentnahmeelements mit dem Probenempfangselement in fluidischem Kontakt bzw. fluidischer Verbindung steht. Somit kann eine rasche Probenaufnahme von beiden Seiten in einem einheitlichen Volumen gewährleistet werden, wobei aufgrund der Kapillarwirkung keine äußere aktive Einwirkung erforderlich ist. Besonders bevorzugt verläuft der Schlitz in Längs- bzw. Stechrichtung eines als Stechelement ausgebildeten Probenentnahmeelements. Denkbar ist aber auch eine Probenaufnahme nach Erzeugung eines Einstichs mittels eines gesonderten Stechapparats. Im Vergleich zu röhrenförmigen oder halboffenen Kapillaren wird durch die offene Schlitzgestaltung auch die Gefahr von Blockierungen durch Gewebebestandteile weiter verringert. Durch die beidseitige Öffnung kann zudem das weitere Probenhandling wesentlich verbessert werden, wobei ein weitgehend totvolumenfreier Transfer erreichbar ist. Für eine auch hinsichtlich der Analyse weitergehende Integration ist das über den Sammelbereich mit Körperflüssigkeit beaufschlagbare, vorzugsweise mit einem Testfeld für einen Analyten in der Körperflüssigkeit versehene Probenempfangselement vorgesehen. Damit lässt sich ein schneller und weitgehend verlustfreier Flüssigkeitstransfer auf das zuvor getrennte Empfangselement realisieren, um die Analyse zu einem definierten Zeitpunkt zu erlauben.

Gemäß einer bevorzugten Ausgestaltung der Erfindung ist die Länge des Längsschlitzes so bemessen, dass in einer Sammelstellung des Probenentnahmeelements der Längsschlitz teilweise innerhalb und teilweise außerhalb des Körperteils ist. Auf diese Weise kann bei der Flüssigkeitsaufnahme eine Entlüftungsfunktion des Schlitzes erreicht werden, und es steht zusätzliches Sammelvolumen zur Verfügung. In dieser Hinsicht ist es vorteilhaft, wenn der Längsschlitz beim Sammeln der Körperflüssigkeit einen in die Haut des Körperteils hineinragenden distalen Aufnahmeabschnitt und einen außerhalb der Haut befindlichen proximalen Entlüftungsabschnitt aufweist.

Vorteilhafterweise besitzt der Längsschlitz eine Länge von 0,5 bis 4 mm, vorzugsweise von 1 bis 2 mm und eine Breite von weniger als 500 µm, vorzugsweise weniger als 100 µm. Weiter ist es von Vorteil, wenn der Längsschlitz im Abstand vorzugsweise von etwa 50 bis 200 µm zu einer das Stechorgan bildenden distalen Spitze des Probenentnahmeelements angeordnet ist.

Um den bei einem Einstich empfundenen Schmerz zu reduzieren und gleichzeitig ein hinreichendes Aufnahmevolumen zu schaffen, kann das Probenentnahmeelement im Bereich des Längsschlitzes einen im Querschnitt verjüngten distalen Schaftabschnitt und einen im Querschnitt erweiterten proximalen Schaftabschnitt aufweisen.

Eine weitere Verbesserung in dieser Richtung wird dadurch erreicht, dass der Längsschlitz in der Sammelstellung des Probenentnahmeelements von dem Probenempfangselement räumlich und/oder fluidisch getrennt und in einer Transferstellung des Probenentnahmeelements mit dem Probenempfangselement zum Transfer von Körperflüssigkeit über eine Seitenöffnung gekoppelt ist.

Hierbei ist es auch von Vorteil, wenn die dem Auslass gegenüberliegende Seitenöffnung des Längsschlitzes in einer Transferstellung an einen Aktuator zum aktiven Transfer der Körperflüssigkeit auf das Probenempfangselement angeschlossen ist.

Vorteilhafterweise wirkt der Aktuator über pneumatische und/oder mechanische Verdrängungsmittel auf die in dem Längsschlitz befindliche Körperflüssigkeit ein, wobei ein mechanischer Aktuator durch eine gegen den Längsschlitz deformierbare Membran gebildet sein kann. Günstig ist es auch, wenn der Aktuator eine an die von dem Probenempfangselement abgewandte Seitenöffnung des Längsschlitzes ankoppelbare Druckluftpassage aufweist.

Für einen möglichst vollständigen Flüssigkeitstransfer ist es vorteilhaft, wenn das Probenempfangselement eine zumindest vergleichbar große oder größere Kapillarattraktion für die Körperflüssigkeit als der Längsschlitz aufweist.

Eine weitere vorteilhafte Ausgestaltung sieht vor, dass das Probenentnahmeelement in einer Führung relativ zu dem Probenempfangselement beweglich gelagert ist. Hierbei ist es aus Schutz- und Hygienegründen vorteilhaft, wenn das Probenempfangselement einen das Probenentnahmeelement aufnehmenden Köcher bildet.

Weitere Gebrauchsvorteile für den Benutzer lassen sich dadurch erzielen, dass mehrere Probenentnahmeelemente in einem ersten Magazin und mehrere Probenempfangselemente in einem zweiten Magazin bevorratet sind, wobei die Magazine als gesonderte Einheiten zur paarweisen Kopplung der Probenentnahmeelemente und Probenempfangselemente miteinander verbindbar sind.

Eine vorteilhafte Realisierungsmöglichkeit besteht darin, dass eine Mehrzahl von Probenentnahmeelementen in einem Magazin, vorzugsweise einem Trommelmagazin axial ausstoßbar angeordnet sind, und dass zugeordnete Probenempfangselemente vorzugsweise in Durchstoßkammern dem Magazin in Ausstoßrichtung vorgeordnet sind.

Gegenstand der Erfindung ist auch ein tragbares Blutanalysegerät zur Aufnahme mindestens einer vorzugsweise als Einmalartikel ausgebildeten erfindungsgemäßen Sammelanordnung.

Die Erfindung umfasst auch ein System zum Analysieren von Körperflüssigkeiten wie Blut, mit einem vorzugsweise mit einem Stechorgan zum Einstechen in ein Körperteil versehenen Probenentnahmeelement, das einen als Kapillare langgestreckten, über Seitenöffnungen beidseitig offenen Längsschlitz als Sammelbereich zum Sammeln von durch einen Einstich erhaltener Körperflüssigkeit aufweist, und mit einem über den Sammelbereich mit Körperflüssigkeit beaufschlagbaren, vorzugsweise mit einem Testfeld für einen Analyten in der Körperflüssigkeit versehenen Probenempfangselement, wobei ein Aktuator zum Transferieren von Körperflüssigkeit von dem Probenentnahmeelement auf das Probenempfangselement vorgesehen ist. Um die Vorteile der beidseitigen Schlitzöffnungen besonders raumsparend auszunutzen, ist es günstig, wenn der Aktuator von einer Seitenöffnung her auf die Körperflüssigkeit in dem Längsschlitz einwirkt, und dass auf der davon abgewandten Seite des Probenentnahmeelements eine Vermessung eines Analyten in der Körperflüssigkeit erfolgt, wobei das Probenentnahmeelement in einer Transferstellung über die von dem Aktuator abgewandte Seitenöffnung mit dem Probenempfangselement in fluidischer Verbindung steht. In einem solchen Instrument mit vorzugsweise disposiblen Elementen zur Flüssigkeitsverarbeitung ist eine vorzugsweise optische Messeinheit zum Erfassen eines Analyten auf dem mit Körperflüssigkeit beaufschlagten Testfeld vorgesehen.

In verfahrensmäßiger Hinsicht wird die eingangs genannte Aufgabe dadurch gelöst, dass in dem Probenentnahmeelement ein beidseitig offener Längsschlitz als Sammelbereich für die Körperflüssigkeit durch Laserschneiden gebildet wird.

Hierbei ist es günstig, wenn die Begrenzungsfläche des Längsschlitzes beim Laserschneiden hydrophiliert wird.

Eine weitere Verbesserung sieht vor, dass die Laserenergie und/oder die Verfahrgeschwindigkeit des Laserstrahls beim Laserschneiden positionsabhängig geregelt wird, so dass unterschiedliche Materialstärken besser berücksichtigt werden können.

Vorteilhafterweise beträgt die Bearbeitungszeit während des Laserschneidens weniger als 2 s, vorzugsweise etwa 1 s.

Auch für eine erhöhte Kapillarwirkung ist es von Vorteil, wenn die Begrenzungskanten des Längsschlitzes mit einem Kantenwinkel von weniger als 100°, vorzugsweise etwa 90° gebildet werden.

Eine weitere vorteilhafte Verfahrensweise sieht vor, dass an dem Probenentnahmeelement ein scharfes Stechorgan durch Schleifen erzeugt wird, so dass das Stechorgan durch mindestens einen Flachschliff begrenzt wird. Hierbei ist es auch von Vorteil, wenn ein Draht oder ein flaches Band als Ausgangsmaterial bearbeitet wird.

Im Folgenden wird die Erfindung anhand der in der Zeichnung schematisch dargestellten Ausführungsbeispiele näher erläutert. Es zeigen
- Fig. 1: ein Handgerät zur Blutzuckermessung in einer perspektivischen Ansicht;
- Fig. 2: ein Stechelement mit einem Längsschlitz zur Blutaufnahme in einer perspektivischen Ansicht;
- Fig. 3: eine integrierte Messanordnung mit Stechelement und Testelement in verschiedenen Prozessschritten in der Perspektive;
- Fig. 4: einen Fig. 3 entsprechenden Messablauf im Axialschnitt;
- Fig. 5: ein Magazin für ein Blutzuckermessgerät in einer Explosionsdarstellung;
- Fig. 6: den Messablauf beim Einsatz des Magazins nach Fig. 5 im Längsschnitt;
- Fig. 7: eine ausschnittsweise Vergrößerung der Fig. 6c.

Die in der Zeichnung dargestellte Anordnung 1 umfasst mindestens ein Probenentnahmeelement 10 mit einem als Längsschlitz 12 ausgebildeten Sammelbereich zum Sammeln von an einer Einstichstelle aus einem Körperteil 14 erhaltener Körperflüssigkeit und ein mit dem Längsschlitz in fluidische Verbindung bringbares Probenempfangselement 16 zum Blutzuckernachweis.

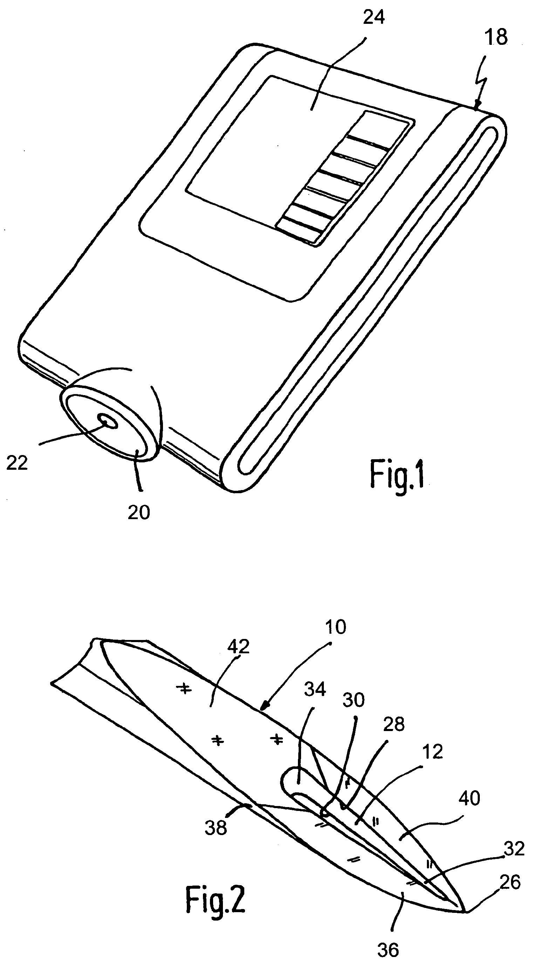

Fig. 1 zeigt ein portables Blutzuckermessgerät 18 für den Einsatz einer solchen Messanordnung 1 für das so genannte "Spot-Monitoring", d.h. die Selbstbestimmung der Blutzuckerkonzentration zu einer gegebenen Zeit durch einen Probanden. Zu diesem Zweck weist das Gerät 18 eine nach innen konische Auflage 20 zur Fingerpositionierung über einer Durchstechöffnung 22 für das Probenentnahme- bzw. Stechelement 10 auf. Die einzelnen Gerätekomponenten werden in einem vollautomatischen Messablauf aktiviert, so dass in Verbindung mit einer in das Gerät eingesetzten, vorzugsweise disposiblen Anordnung 1 der Benutzer schließlich auf einer Anzeige 24 einen Messwert über seinen momentanen Blutglukosespiegel erhält, ohne dass eine aufwändige Handhabung erforderlich wäre. Generell können mit einem solchen System die Messungen auch an anderen Körperteilen, beispielsweise im weniger schmerzempfindlichen Arm- oder Bauchbereich vorgenommen werden, wobei als Körperflüssigkeit für die Probennahme neben Kapillarblut aus der Haut auch Gewebeflüssigkeit oder Mischungen davon in Frage kommen.

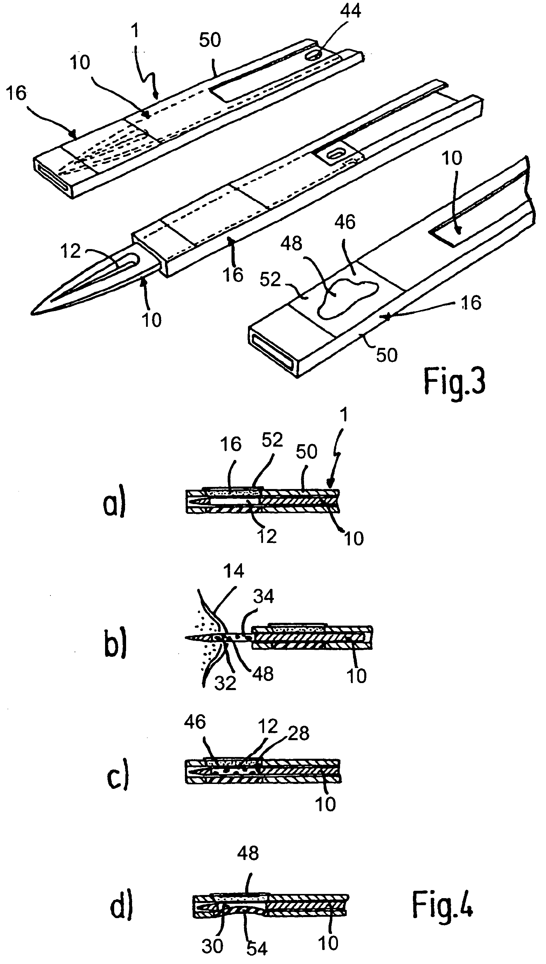

Wie aus Fig. 2 zu ersehen, erstreckt sich der Längsschlitz 12 in Längsrichtung des schaftförmigen Stechelements 10, beispielsweise über eine Länge von 1 bis 2 mm bei einer Breite von 100 bis 200 µm. Der Abstand zu einer das Stechorgan bildenden Spitze 26 kann etwa der Schlitzbreite entsprechen. Auf diese Weise wird eine Kapillaraufnahme gebildet, in welche die beim Einstich in dem Körperteil 14 (Fig. 4) zugängliche Körperflüssigkeit aufgrund von Kapillarwirkung selbsttätig einströmt.

Die Flüssigkeitsaufnahme erfolgt beidseitig über die einander gegenüberliegenden Seitenöffnungen 28, 30 des Längsschlitzes 12. Hierbei ist die Schlitzlänge in Stechrichtung so bemessen, dass ein distaler Schlitzabschnitt 32 in die Haut des Körperteils 14 hineinragt und ein proximaler Schlitzabschnitt 34 sich außerhalb der Haut befindet. Zur Schmerzverringerung kann der in den Körper eingreifende distale Schaftabschnitt 36 "dünn", d.h. im Querschnitt verjüngt ausgeführt sein, während der außerhalb des Körpers verbleibende proximale Schaftabschnitt 38 demgegenüber einen erweiterten Querschnitt bzw. eine vergleichsweise größere Dicke aufweist, um eine hinreichend große Flüssigkeitsmenge sammeln zu können. Beispielsweise kann die Flüssigkeitsmenge entsprechend dem Schlitzvolumen 10 bis 20 Nanoliter betragen, wobei das im Körper befindliche Teilvolumen des Längsschlitzes 12 beispielsweise einen Volumenanteil von 20% umfasst.

Bei der Herstellung solcher Stechelemente 10 wird zunächst ein Draht als Ausgangsmaterial in einer definierten Ausrichtung durch Schleifprozesse bearbeitet, wobei die unterschiedlich abgewinkelten Schliffflächen 40, 42 erzeugt werden. Sodann wird in dieser Flächenstruktur zweckmäßig in derselben Bearbeitungsstation unter Beibehaltung der Drehausrichtung mittels eines geeignet positionierten Lasers der Längsschlitz 12 eingebracht. Ein wichtiges Produktionsdetail liegt darin, dass die Laserenergie und/oder die Verfahrgeschwindigkeit des Laserstrahls in Abhängigkeit von der Schneidposition geregelt werden; damit lassen sich zulaufende Öffnungen gestalten oder auch unterschiedliche Materialdicken kompensieren.

Für den Schlitzdurchbruch sind nur wenige Bewegungen des Laserstrahls nötig, und die Bearbeitungszeit kann für eine Massenfertigung ausreichend kurz gehalten werden, beispielsweise im Bereich von 1 sec. Ebenso können die mechanischen Toleranzen gering gehalten werden, beispielsweise im Bereich von 10 µm, wobei Wandsteilheiten von nahezu 90° erreichbar sind. Zudem können durch geeignete Laserbehandlung insbesondere unter Schutz- bzw. Spezialgasen für eine Beschichtung aktivierte und/oder hydrophile Oberflächen geschaffen werden, die sich gegebenenfalls durch eine physikalische oder nasschemische Nachbehandlung weiter verbessern lassen.

Die solchermaßen geschaffenen Lanzetten 10 können orientiert in einen Kunststoffhalter eingebracht werden. Dieser kann z.B. von einer Rolle kommen, und durch Einklipsen, Warmverformen etc. kann die Verbindung zur Lanzette lagerichtig erfolgen. Der Kunststoffhalter kann ein Kopplungsstück aufweisen, damit sich die Lanzetten im Gerät 18 greifen und bewegen lassen.

Fig. 3 zeigt eine Einheit aus Probenentnahmeelement (Stechelement 10) und Probenempfangselement 16. Die Probenentnahme erfolgt durch hin- und hergehende Stechbewegung des Stechelements 10, wobei an dem proximalen Schaftteil 44 ein geeigneter Antrieb formschlüssig angekoppelt wird. Das während des Stechvorgangs gerätefest gehaltene Probenempfangselement 16 ist Teil einer als Köcher ausgebildeten Schubführung für das Stechelement 10 und weist ein Testfeld 46 zum Aufnehmen der zuvor in dem Längsschlitz 12 gesammelten Körperflüssigkeit 48 auf. Der als Linearführung wirkende Köcher umschließt also das Stechelement in einer rechteckig-flachen Konfiguration, wobei die Breitseiten den Seitenöffnungen 28, 30 des Längsschlitzes 12 zugewandt sind. Neben einem vorteilhaften Flüssigkeitstransfer wird so ein Schutz des Stechelements 10 und eine hygienische Entsorgung sichergestellt.

Das Stechelement 10 erfüllt somit eine Zubringerfunktion als "Shuttle" bei der Probengewinnung, während der eigentliche Nachweis des Analyten auf dem mit dem Körperteil 14 nicht in Berührung kommenden Probenempfangselement 16 erfolgt. Dieses kann auch als Komponente eines eine Mehrzahl von abgeteilten Kompartimenten 50 umfassenden Magazins vorgesehen sein, welches sich als Verbrauchsartikel in das Gerät 18 einsetzen und mit den verbrauchten Stechelementen wieder entsorgen lässt. Der Glucosenachweis auf dem Testfeld bzw. Reagenzträger 46 kann photometrisch über Reflexion durch das transparente Deckfenster 52 hindurch erfolgen, so dass die Blutflüssigkeit 48 in dem Kompartiment 50 von den Geräteteilen hygienisch getrennt bleibt. Denkbar sind auch andere Nachweistechniken, beispielsweise über Fluoreszenz oder elektrochemische Erfassung.

In Fig. 4 sind die wesentlichen Schritte des Probentransfers nochmals näher veranschaulicht. In der Ausgangsstellung (4a) befindet sich das Stechelement 10 geschützt in dem Magazinfach 50, welches frontseitig durch eine Durchstechfolie versiegelt sein kann. Die Einstichbewegung in das Körperteil 14 (beispielsweise eine Fingerkuppe) erfolgt aus Schmerzgründen möglichst rasch und bewegungsoptimiert, wobei gegebenenfalls aus der maximalen Einstechtiefe eine leicht zurückgezogene Sammelposition angesteuert werden kann. Vorteilhafterweise ragt für den Sammelvorgang entsprechend (4b) der distale Schlitzabschnitt 32 in das Hautinnere, während der proximale Schlitzabschnitt 34 außerhalb davon eine Entlüftungsfunktion erfüllt. Somit kann die im Einstichkanal gewonnene Blutflüssigkeit in kurzer Sammelzeit beidseitig in den Sammelschlitz 12 einströmen und effizient das gesamte Schlitzvolumen füllen. In der Sammelstellung ist der Schlitz 12 von dem Probenempfangselement 16 räumlich so getrennt, dass keine fluidische Verbindung dazwischen besteht und die Nachweisreagenzien nicht in das Körperinnere gelangen können. Dies erlaubt es auch, den Nachweisvorgang gezielt zu starten und den Messverlauf als solchen auszuwerten. Zu diesem Zweck wird entsprechend Fig. 4c) das Stechelement 10 wieder zurückgezogen, bis die obere Seitenöffnung 28 des Schlitzes 12 unter das Testfeld 46 gelangt. Dieses kann beispielsweise durch eine Vliesstruktur eine größere Kapillarattraktivität als der Schlitz 12 für einen selbsttätigen Transfer des aufgenommenen Blutes besitzen. Bevorzugt wird jedoch der Flüssigkeitstransfer unterstützt, indem gemäß Fig. 4d) an der unteren Schlitzöffnung 30 eine Membran 54 als Verdrängungsmittel für die gesammelte Flüssigkeit eingedrückt wird. Der Fluidtransfer erfolgt also über die gesamte Schlitzlänge mit kurzem Weg quer zur Stechrichtung des Stechelements 10.

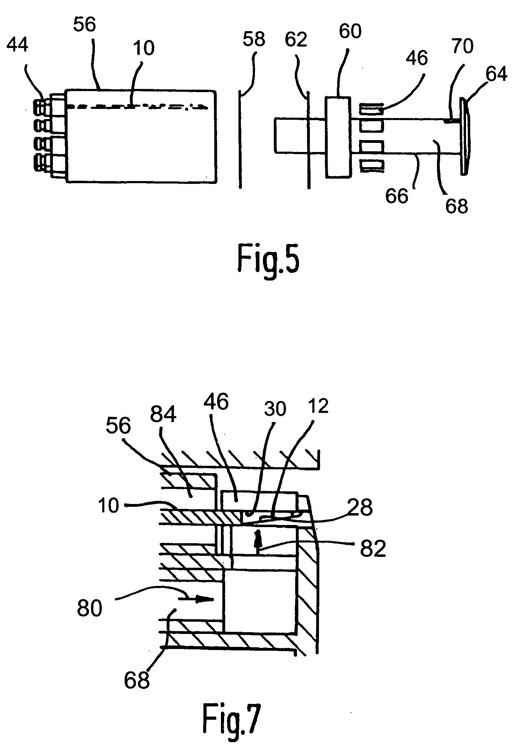

Auch die in Fig. 5 bis 7 gezeigte Ausführungsform verwirklicht dieses Prinzip, wobei eine spezielle Magazinierung und Aktuierung vorgesehen ist. Gemäß Fig. 5 sind die Stechelemente 10 in einer Lanzettentrommel 56 magaziniert, an deren hinterer Stirnseite die Kopplungsstücke 44 für die Antriebskopplung abstehen, während die vordere (distale) Stirnseite durch eine Durchstechfolie 58 versiegelt wird. Eine gesonderte Teststreifentrommel 60 kann über Einschubschlitze mit Teststreifen 46 bestückt werden, welche dann durch eine Folie 62 und einen Stirndeckel 64 gegenüber der Umgebung insbesondere gegen Feuchtigkeitszutritt abgeschirmt werden. Der Stirndeckel 64 sitzt auf einer Trommelhohlachse 66, welche zugleich eine Luftpassage 68 für eine pneumatische Aktuierung über die mantelseitige Blasöffnung 70 bildet. Durch die gesonderte Magazinierung kann unterschiedlichen Anforderungen Sorge getragen werden. Die mit dem Körper in Kontakt kommenden Stechelemente 10 lassen sich in der Lanzettentrommel 56 zweckmäßig durch energiereiche Strahlung sterilisieren, während die strahlungsempfindliche Testchemie unabhängig davon in der Teststreifentrommel 60 gegen äußere Einflüsse geschützt bleibt und beim Stechvorgang auch nicht in Körperberührung gelangt.

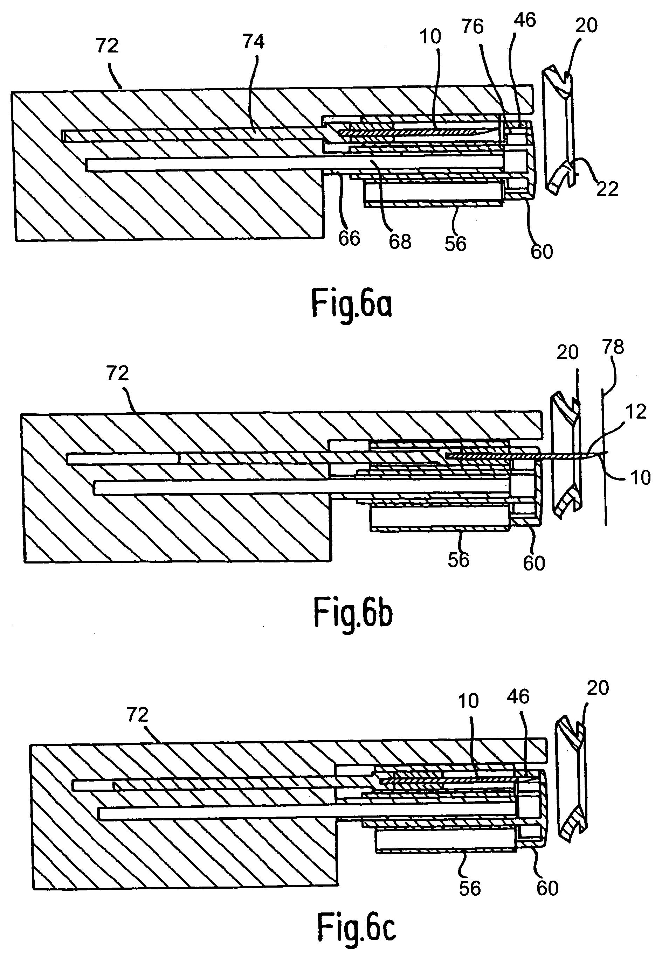

Wie aus Fig. 6 ersichtlich, sind die Magazine 56, 60 im Einbauzustand auf der Trommelachse 66 axial gekoppelt, so dass jeweils ein Stechelement 10 und ein Teststreifen 46 einander zugeordnet sind. Eine Magazinaufnahme 72 innerhalb des Geräts 18 ermöglicht die Druckluftbeaufschlagung der Luftpassage 68 und den Stechantrieb des jeweils an einen Antriebsstößel 74 angekoppelten Stechelements 10 in koaxialer Ausrichtung zu dem Fingerkonus 20 (Fig. 6a). Beim Stechvorschub durchstößt das betätigte Stechelement 10 die vorgelagerte Kammer 76 des zugeordneten Teststreifens 46 und erreicht durch die Konusöffnung 22 hindurch die maximale Vorschubposition (Linie 78 in Fig. 6b) bei einem zurückgelegten Weg von etwa 10 mm. Die Blutaufnahme über den Schlitz 12 erfolgt dann wie oben beschrieben. Anschließend wird bei der Rückbewegung des Stechelements 10 die in Fig. 6c gezeigte Transferposition angefahren, in welcher der Schlitz 10 in seiner Durchlassrichtung mit dem Teststreifen 46 fluchtet.

Wie am besten aus dem vergrößerten Ausschnitt gemäß Fig. 7 ersichtlich, ist in der Transferposition eine gezielte Übergabe der gesammelten Flüssigkeit auf den Teststreifen 46 möglich. Zu diesem Zweck wird über die Luftpassage 68 in Richtung der Pfeile 80, 82 ein Luftstoß ausgelöst, welcher den Schlitz 12 an der zugewandten Seitenöffnung 28 beaufschlagt, so dass die Blutflüssigkeit über die gegenüberliegende Seitenöffnung 30 auf den Teststreifen 46 verdrängt und dort vermessen wird. Der Glucosenachweis kann dann über eine nicht gezeigte Geräteoptik reflektometrisch durchgeführt werden. Anschließend wird das kontaminierte Stechelement 10 in die Aufnahmekammer 84 des Trommelmagazins 56 vollständig zurückgezogen und durch Trommeldrehung das nächste Funktionspaar 10, 46 in Bereitschaft gebracht.

## Patentansprüche

1. Anordnung zum Aufnehmen von Körperflüssigkeiten wie Blut, mit einem vorzugsweise mit einem Stechorgan zum Einstechen in ein Körperteil (14) versehenen Probenentnahmeelement (10), das einen Sammelbereich (12) zum Sammeln von durch einen Einstich erhaltener Körperflüssigkeit aufweist, wobei der Sammelbereich durch einen als Kapillare langgestreckten, über Seitenöffnungen (28,30) an dem Probenentnahmeelement (10) beidseitig offenen Längsschlitz (12) gebildet ist, und mit einem über den Sammelbereich (12) mit Körperflüssigkeit beaufschlagbaren, vorzugsweise mit einem Testfeld (46) für einen Analyten in der Körperflüssigkeit versehenen Probenempfangselement (16), **dadurch gekennzeichnet, dass** das Probenentnahmeelement (10) in einer Führung (50) relativ zu dem Probenempfangselement (16) hin- und hergehend beweglich gelagert ist, wobei der Längsschlitz (12) in einer Sammelstellung des Probenentnahmeelements (10) von dem Probenempfangselement (16) räumlich so getrennt ist, dass keine fluidische Verbindung dazwischen besteht, und in einer Transferstellung des Probenentnahmeelements (10) mit dem Probenempfangselement (16) über eine Seitenöffnung (28,30) als Auslass des Längsschlitzes (12) in fluidischem Kontakt steht.

2. Anordnung nach Anspruch 1, **dadurch gekennzeichnet, dass** die Länge des Längsschlitzes (12) so bemessen ist, dass in der Sammelstellung des Probenentnahmeelements (10) der Längsschlitz (12) teilweise innerhalb und teilweise außerhalb des Körperteils (14) ist.

3. Anordnung nach Anspruch 1 oder 2, **dadurch gekennzeichnet, dass** der Längsschlitz (12) beim Sammeln der Körperflüssigkeit einen in die Haut des Körperteils (14) hineinragenden distalen Aufnahmeabschnitt (32) und einen außerhalb der Haut befindlichen proximalen Entlüftungsabschnitt (34) aufweist.

4. Anordnung nach einem der Ansprüche 1 bis 3, **dadurch gekennzeichnet, dass** der Längsschlitz (12) eine Länge von 0,5 bis 4 mm, vorzugsweise von 1 bis 2 mm und eine Breite von weniger als 500 µm, vorzugsweise weniger als 100 µm aufweist.

5. Anordnung nach einem der Ansprüche 1 bis 4, **dadurch gekennzeichnet, dass** der Längsschlitz (12) im Abstand vorzugsweise von etwa 50 bis 200 µm zu einer das Stechorgan bildenden distalen Spitze (26) des Probenentnahmeelements (10) angeordnet ist.

6. Anordnung nach einem der Ansprüche 1 bis 5, **dadurch gekennzeichnet, dass** das Probenentnahmeelement (10) im Bereich des Längsschlitzes (12) einen im Querschnitt verjüngten distalen Schaftabschnitt (40) und einen im Querschnitt erweiterten proximalen Schaftabschnitt (42) aufweist.

7. Anordnung nach einem der Ansprüche 1 bis 6, **dadurch gekennzeichnet, dass** eine Seitenöffnung (28,30) des Längsschlitzes (12) in der Transferstellung an einen Aktuator (54;68) zum Transfer der Körperflüssigkeit auf das Probenempfangselement (16) angeschlossen ist.

8. Anordnung nach Anspruch 7, **dadurch gekennzeichnet, dass** der Aktuator (54;68) über pneumatische und/oder mechanische Verdrängungsmittel auf die in dem Längsschlitz (12) befindliche Körperflüssigkeit einwirkt.

9. Anordnung nach Anspruch 7 oder 8, **dadurch gekennzeichnet, dass** der Aktuator durch eine gegen den Längsschlitz (12) deformierbare Membran (54) gebildet ist.

10. Anordnung nach einem der Ansprüche 7 bis 9, **dadurch gekennzeichnet, dass** der Aktuator eine an eine Seitenöffnung (28,30) des Längsschlitzes ankoppelbare Druckluftpassage (68) aufweist.

11. Anordnung nach einem der Ansprüche 1 bis 10, **dadurch gekennzeichnet, dass** das Probenempfangselement (16) eine größere Kapillarattraktion für die Körperflüssigkeit als der Längsschlitz (12) aufweist.

12. Anordnung nach einem der Ansprüche 1 bis 11, **dadurch gekennzeichnet, dass** das Probenentnahmeelement (10) in einer Führung (50) relativ zu dem Probenempfangselement (16) beweglich gelagert ist.

13. Anordnung nach einem der Ansprüche 1 bis 12, **dadurch gekennzeichnet, dass** das Probenempfangselement (16) einen das Probenentnahmeelement (10) aufnehmenden Köcher bildet.

14. Anordnung nach einem der Ansprüche 1 bis 13, **dadurch gekennzeichnet, dass** mehrere Probenentnahmeelemente (10) in einem ersten Magazin (56) und mehrere Probenempfangselemente (16;46) in einem zweiten Magazin (60) bevorratet sind, wobei die Magazine (56,60) als gesonderte Einheiten zur paarweisen Kopplung der Probenentnahmeelemente und Probenempfangselemente miteinander verbindbar sind.

15. Anordnung nach einem der Ansprüche 1 bis 14, **dadurch gekennzeichnet, dass** eine Mehrzahl von Probenentnahmeelementen (10) in einem Magazin (56), vorzugsweise einem Trommelmagazin axial ausstoßbar angeordnet sind, und dass zugeordnete Probenempfangselemente (46) vorzugsweise in Durchstoßkammern (76) dem Magazin (56) in Ausstoßrichtung vorgeordnet sind.

16. Tragbares Blutanalysegerät mit mindestens einer vorzugsweise als Einmalartikel ausgebildeten Anordnung (1) nach einem der vorhergehenden Ansprüche.

17. System zum Analysieren von Körperflüssigkeiten wie Blut, mit einer Anordnung (1) nach einem der vorhergehenden Ansprüche **dadurch gekennzeichnet, dass** ein Aktuator (54;68) zum Transferieren von Körperflüssigkeit von dem Probenentnahmeelement (10) auf das Probenempfangselement (16) vorgesehen ist.

18. System nach Anspruch 17, **dadurch gekennzeichnet, dass** der Aktuator (54;68) von einer Seitenöffnung (28,30) her auf die Körperflüssigkeit in dem Längsschlitz einwirkt, und dass auf der davon abgewandten Seite des Probenentnahmeelements (10) eine Vermessung eines Analyten in der Körperflüssigkeit erfolgt.

19. System nach Anspruch 18, **dadurch gekennzeichnet, dass** das Probenentnahmeelement (10) in der Transferstellung über die von dem Aktuator (54;68) abgewandte Seitenöffnung mit dem Probenempfangselement (16) in fluidischer Verbindung steht.

20. System nach einem der Ansprüche 17 bis 19, **gekennzeichnet durch** eine vorzugsweise optische Messeinheit zum Erfassen eines Analyten auf dem mit Körperflüssigkeit beaufschlagten Testfeld (46).

## Claims

1. Assembly for receiving body fluids such as blood comprising a sampling element (10) which is preferably provided with a lancing member for insertion into a body part (14) and has a collecting area (12) for collecting body fluid obtained by a puncture, wherein the collecting area is formed by a longitudinal slot (12) which is elongated as a capillary and is open on both sides via side openings (28, 30) on the sampling element (10), and comprising a sample receiving element (16) to which body fluid can be applied via the collecting area (12) and preferably provided with a test field (46) for an analyte in the body fluid, **characterized in that** the sampling element (10) is movably mounted to reciprocate in a guide (50) relative to the sample receiving element (16), wherein the longitudinal slot (12) is spatially separated from the sample receiving element (16) in a collecting position of the sampling element (10) in such a manner that there is no fluidic connection between them and is in fluidic contact via a side opening (28, 30) as an outlet of the longitudinal slot (12) with the sample receiving element (16) in a transfer position of the sampling element (10).

2. Assembly according to claim 1, **characterized in that** the length of the longitudinal slot (12) is such that the longitudinal slot (12) is partially within and partially outside the body part (14) in the collecting position of the sampling element (10).

3. Assembly according to claim 1 or 2, **characterized in that** the longitudinal slot (12) has a distal receiving section (32) protruding into the skin of the body part (14) and a proximal venting section (34) located outside the skin when the body fluid is collected.

4. Assembly according to one of the claims 1 to 3, **characterized in that** the longitudinal slot (12) has a length of 0.5 to 4 mm, preferably of 1 to 2 mm and a width of less than 500 µm, preferably less than 100 µm.

5. Assembly according to one of the claims 1 to 4, **characterized in that** the longitudinal slot (12) is arranged at a distance of preferably about 50 to 200 µm from a distal tip (26) of the sampling element (10) which forms the lancing member.

6. Assembly according to one of the claims 1 to 5, **characterized in that** the sampling element (10) has a distal shaft section (40) with a tapered cross-section in the area of the longitudinal slot (12) and a proximal shaft section (42) with a widened cross-section.

7. Assembly according to one of the claims 1 to 6, **characterized in that** a side opening (28, 30) of the longitudinal slot (12) is connected in the transfer position to an actuator (54; 68) in order to transfer body fluid onto the sample receiving element (16).

8. Assembly according to claim 7, **characterized in that** the actuator (54; 68) acts on the body fluid in the longitudinal slot (12) by means of pneumatic and/or mechanical displacement means.

9. Assembly according to claim 7 or 8, **characterized in that** the actuator is formed by a membrane (54) that can be deformed against the longitudinal slot (12).

10. Assembly according to one of the claims 7 to 9, **characterized in that** the actuator has a passage (68) for compressed air that can be coupled to a side opening (28, 30) of the longitudinal slot.

11. Assembly according to one of the claims 1 to 10, **characterized in that** the sample receiving element (16) has a larger capillary attraction for the body fluid than the longitudinal slot (12).

12. Assembly according to one of the claims 1 to 11, **characterized in that** the sampling element (10) is movably mounted in a guide (50) relative to the sample receiving element (16).

13. Assembly according to one of the claims 1 to 12, **characterized in that** the sample receiving element (16) forms a case which receives the sampling element (10).

14. Assembly according to one of the claims 1 to 13, **characterized in that** several sampling elements (10) are stored in a first magazine (56) and several sample receiving elements (16; 46) are stored in a second magazine (60), wherein the magazines (56, 60) as separate units can be connected together in order to couple the sampling elements and sample receiving elements in pairs.

15. Assembly according to one of the claims 1 to 14, **characterized in that** a plurality of sampling elements (10) are arranged in a magazine (56), preferably in a drum magazine such that they can be ejected axially and that their associated sample receiving elements (46) preferably arranged in push-through chambers (76) are arranged in front of the magazine (56) in the direction of ejection.

16. Portable blood analyser comprising at least one assembly (1) according to one of the previous claims preferably in the form of a single-use article.

17. System for analysing body fluids such as blood comprising an assembly (1) according to one of the previous claims, **characterized in that** an actuator (54; 68) is provided for transferring body fluid from the sampling element (10) onto the sample receiving element (16).

18. System according to claim 17, **characterized in that** the actuator (54; 68) acts from a side opening (28, 30) on the body fluid in the longitudinal slot and that an analyte in the body fluid is measured on the opposing side of the sampling element (10).

19. System according to claim 18, **characterized in that** in a transfer position, the sampling element (10) is in fluidic contact with the sample receiving element (16) via the side opening facing away from the actuator (54; 68).

20. System according to one of the claims 17 to 19, **characterized in that** a preferably optical measuring unit is provided to detect an analyte on the test field (46) to which body fluid has been applied.

## Revendications

1. Agencement destiné à recevoir des fluides corporels comme du sang, avec un élément de prélèvement d'échantillon (10) muni de manière préférée d'un organe de piqûre destiné à être piqué dans une partie corporelle (14), ledit élément présentant un secteur de collecte (12) destiné à une collecte de fluide corporel obtenu grâce à une piqûre, dans lequel le secteur de collecte est formé d'une fente longitudinale (12), allongée en forme de capillaire, et ouverte de part et d'autre au niveau de l'élément de prélèvement d'échantillon (10) grâce à des ouvertures latérales (28, 30), et avec un élément de réception d'échantillon (16) pouvant être soumis à un fluide corporel grâce au secteur de collecte (12), de manière préférée muni d'un champ de test (46) pour un analyte présent dans le fluide corporel, **caractérisé en ce que** l'élément de prélèvement d'échantillon (10) est monté mobile d'avant en arrière par rapport à l'élément de réception d'échantillon (16) dans un guide (50), dans lequel la fente longitudinale (12), dans une position de collecte de l'élément de prélèvement d'échantillon (10), est séparée spatialement de l'élément de réception d'échantillon (16) de telle manière qu'aucune liaison fluidique n'existe entre ceux-ci, et, dans une positon de transfert de l'élément de prélèvement d'échantillon (10), est en contact fluidique avec l'élément de réception d'échantillon (16) grâce à une ouverture latérale (28, 30) faisant office de sortie de la fente longitudinale (12).

2. Agencement selon la revendication 1, **caractérisé en ce que** la longueur de la fente longitudinale (12) est mesurée de telle manière que, dans la position de collecte de l'élément de prélèvement d'échantillon (10), la fente longitudinale (12) est partiellement à l'intérieur et partiellement à l'extérieur de la partie corporelle (14).

3. Agencement selon la revendication 1 ou 2, **caractérisé en ce que** la fente longitudinale (12) présente, lors de la collecte du fluide corporel, une section de prise (32) distale rentrant dans la peau de la partie corporelle (14) et une section d'aération (34) proximale se trouvant à l'extérieur de la peau.

4. Agencement selon l'une quelconque des revendications 1 à 3, **caractérisé en ce que** la fente longitudinale (12) présente une longueur comprise entre 0,5 et 4 mm, de manière préférée entre 1 et 2 mm et une largeur inférieure à 500 µm, de manière préférée inférieure à 100 µm.

5. Agencement selon l'une quelconque des revendications 1 à 4, **caractérisé en ce que** la fente longitudinale (12) est agencée avec un écart compris de manière préférée entre environ 50 et 200 µm par rapport à une pointe (26) distale, formant l'organe de piqûre, de l'élément de prélèvement d'échantillon (10).

6. Agencement selon l'une quelconque des revendications 1 à 5, **caractérisé en ce que** l'élément de prélèvement d'échantillon (10) présente, dans le secteur de la fente longitudinale (12) une section formant lancette (40) distale convergent en coupe transversale et une section formant lancette (42) proximale élargissant en coupe transversale.

7. Agencement selon l'une quelconque des revendications 1 à 6, **caractérisé en ce qu'**une ouverture latérale (28, 30) de la fente longitudinale (12) est raccordée, dans la position de transfert, à un actionneur (54 ; 68) destiné au transfert du fluide corporel vers l'élément de réception d'échantillon (16).

8. Agencement selon la revendication 7, **caractérisé en ce que** l'actionneur (54 ; 68) agit sur le fluide corporel présent dans la fente longitudinale (12) grâce à un moyen de déplacement pneumatique et/ou mécanique.

9. Agencement selon la revendication 7 ou 8, **caractérisé en ce que** l'actionneur est formé d'une membrane (54) pouvant être déformée contre la fente longitudinale (12).

10. Agencement selon l'une quelconque des revendications 7 à 9, **caractérisé en ce que** l'actionneur présente un passage d'air comprimé (68) pouvant être couplé à une ouverture latérale (28, 30) de la fente longitudinale.

11. Agencement selon l'une quelconque des revendications 1 à 10, **caractérisé en ce que** l'élément de réception d'échantillon (16) présente une attraction capillaire envers le fluide corporel qui est supérieure à celle de la fente longitudinale (12).

12. Agencement selon l'une quelconque des revendications 1 à 11, **caractérisé en ce que** l'élément de prélèvement d'échantillon (10) est monté mobile par rapport à l'élément de réception d'échantillon (16) dans un guide (50).

13. Agencement selon l'une quelconque des revendications 1 à 12, **caractérisé en ce que** l'élément de réception d'échantillon (16) forme un réceptacle accueillant l'élément de prélèvement d'échantillon (10).

14. Agencement selon l'une quelconque des revendications 1 à 13, **caractérisé en ce que** plusieurs éléments de prélèvement d'échantillon (10) sont empilés dans un premier chargeur (56) et plusieurs éléments de réception d'échantillon (16 ; 46) sont empilés dans un deuxième chargeur (60), dans lequel les chargeurs (56, 60) peuvent être reliés l'un à l'autre sous forme d'unités séparées en vue d'un couplage par paire des éléments de prélèvement d'échantillon et des éléments de réception d'échantillon.

15. Agencement selon l'une quelconque des revendications 1 à 14, **caractérisé en ce qu'**une pluralité d'éléments de réception d'échantillon (10) sont agencés axialement éjectables dans un chargeur (56), de manière préférée un chargeur tambour, et les éléments de réception d'échantillon (46) associés sont de manière préférée pré-rangés dans le sens d'éjection dans des chambres de à percer (76) du chargeur (56).

16. Appareil d'analyse sanguine portatif avec au moins un agencement (1) selon l'une quelconque des revendications précédentes, réalisé de manière préférée sous la forme d'un article à usage unique.

17. Système destiné à analyser des fluides corporels comme du sang, avec un agencement (1) selon l'une quelconque des revendications précédentes, **caractérisé en ce qu'**un actionneur (54 ; 68) destiné au transfert d'un fluide corporel depuis un élément de prélèvement d'échantillon (10) vers l'élément de réception d'échantillon (16) est prévu.

18. Système selon la revendication 17, **caractérisé en ce que** l'actionneur (54 ; 68) agit depuis une ouverture latérale (28, 30) sur le fluide corporel présent dans la fente longitudinale, et une mesure d'un analyte présent dans le fluide corporel a lieu du côté de l'élément de prélèvement d'échantillon (10) qui est détourné de ladite ouverture.

19. Système selon la revendication 18, **caractérisé en ce que** l'élément de prélèvement d'échantillon (10), dans la position de transfert, est en liaison fluidique avec l'élément de réception d'échantillon (16) grâce à l'ouverture latérale qui est détournée de l'actionneur (54 ; 68).

20. Système selon l'une quelconque des revendications 17 à 19, **caractérisé par** une unité de mesure, de préférence optique, destinée à détecter un analyte présent sur le champ de test (46) soumis à un fluide corporel.
